# EUROPEAN PATENT SPECIFICATION

(11) **EP 1 197 178 B1**
(45) Date of publication and mention of the grant of the patent: **09.07.2008**
(21) Application number: 01308591.5
(22) Date of filing: 08.10.2001
(51) Int. Cl.: A61B 5/00

(54) **Mobile clinical information system**
Mobiles klinisches Informationssystem
Système d'information clinique mobile

(30) Priority: 12.10.2000 US 689374
(43) Date of publication of application: 17.04.2002
(73) Proprietor: GE Medical Systems Information Technologies, Inc., Milwaukee, Wisconsin 53223-3293 (US)
(72) Inventor: Brinsfield, James W., Mequon, Wisconsin 53097 (US); Hutchinson, George M., Brookfield, Wisconsin 53045 (US)
(74) Representative: Goode, Ian Roy

(56) References cited:
- US-A- 4 916 441
- US-A- 5 687 717
- US-A- 6 057 758

## Description

The present invention pertains to clinical information systems and more specifically, to a two-way, wireless clinical patient information monitoring system and a portable patient monitor.

Clinical patient monitoring systems generally consist of individual patient monitoring terminals connected to a centralized monitoring system staffed by a nurse or clinician. The centralized character of these systems allows a small number of caregivers to monitor a large number of patients. The patient monitor terminals typically stationed in the patients' rooms register activity such as heart rate, ECG, respiratory patterns, and other pertinent signs. In addition, drug infusion devices stationed by the patient deliver regulated dosages as prescribed and programmed by doctors and nurses. For bedside monitoring, these devices work adequately. However, patient mobility is hindered and becomes a hazard when transporting the bulky, inelegant bedside patient monitoring systems.

Another drawback of present clinical patient monitoring systems is that, while providing increased efficiency compared to earlier methods, nurses and other caregivers are still very dependent upon the information displayed at the patient's bedside. At the central nurses' station the monitoring system registers alarms and notifies health care providers when attention is required for a particular patient. However, without proceeding to the patient's room, a caregiver cannot discern the nature or seriousness of the alert. Some more recent systems have incorporated remote patient monitoring through the use of laptop computers, but that has not eliminated the necessity of accessing the bedside equipment manually to adjust alarm parameters or change drug administration. An added burden placed upon the health care provider by using laptop systems is the cumbersome nature of carrying a laptop throughout the day. For example, placing the laptops on rolling carts eliminates the physical burden of carrying the device, but simultaneously eliminates the flexible nature of the mobile system initially envisioned.

Further inhibiting healthcare dispensation and endangering patient welfare is the risk of incorrect drug and dosage administration. Within existing systems, patient data such as prescription information and test results are not readily available in a real time format to on-the-scene health care providers. This problem creates the need for a solution that decreases the likelihood that a health care provider could deliver an erroneous prescription or dosage. A real time connection between prescribed data on file and the delivered quantity, in addition to test results from labs, would considerably enhance the quality and efficiency of the health care provided.

US 5,687,717 discloses a patient monitoring system that includes one or more chassis, a plurality of patient care modules associated with the chassis, and a portable computer for communicating with, and controlling the modules. Each module is fully operational in either an independent or a dependent mode. In both modes, the chassis continuously polls the module for patient data collected by the module. In a dependent mode, the module is physically received by, and powered from, the chassis, and sends patient data to a computer in the chassis for storage therein. The system is arranged in a networked manner, with each chassis and each portable computer being a node on the network. The portable computer is adapted to set or modify module control parameters.

US 4,916,441 discloses a handheld pocket terminal having a display screen and a bar code reader.

US 6,057,758 discloses a system for monitoring a physiological condition of a patient that includes a primary station and a portable station. The primary station includes a transmitter configured to transmit a signal, which represents a physiological condition of the patient, via a wireless communication link. An alarm exhibitor may be configured to exhibit the alarm indication in response to an alarm signal received, via the wireless communication link, from the primary station. The portable station may include a transmitter adapted to communicate, via the wireless communication link, with the primary station to permit the user to respond to the alarm indication.

There is therefore a need for a two-way clinical patient information system. Addressing additional needs such as real time patient information, pharmacological data, and lab results, a two-way system would furnish caregivers with an effectual manner to deliver health care. For instance, a two-way patient monitoring device could permit a nurse to monitor real time patient vital signs, as well as change care parameters such as alarm status, all from a remote location, therefore saving time and energy. Scanning and comparing patient bar codes with prescription bar codes would prevent drug mishandling, and access to recent lab results would reduce the time needed for care decisions. Combining two-way communications ability with a wireless patient monitoring system answers the need for comprehensive, efficient, and accurate health care administration.

The present invention provides two-way, remote, mobile clinical care to patients within a health care facility by health care providers utilizing portable patient monitoring devices of a clinical patient information management system that solves the aforementioned problems.

In accordance with one aspect of the present invention, there is provided wireless bi-directional portable patient monitor that is transportable on a health care provider for extended periods, the portable patient monitor comprising: a communication interface to receive patient data from a wireless local area network (WLAN) within a medical care facility and transmit care parameters as needed to the WLAN in response thereto; a processor connected to the communication interface to process the patient data and the care parameters; a display connected to the processor to display the processed patient data in human discernable form; and an input device connected to the processor to allow a change in the care parameters by a health care provider

In accordance with another aspect of the invention, there is provided a wireless clinical information management system comprising a portable patient monitor of the first aspect that decentralizes patient monitoring by networking information and health care devices through an Ethernet. These devices include life support systems such as ventilators and infusion pumps, along with pharmacy databases, laboratory reports, central patient monitors, telemetry devices, and portable patient monitoring devices. The clinical information management system further involves wireless characteristics through a plurality of wireless LAN access points coupled to a server that process patient telemetry data and PPM instructions.

The portable patient monitor can include a personal data assistant (PDA) that optionally provides PDA functions to a health care provider. A few of the PDA functions accessible to the health care provider consist of a scheduler, reminders, to-do lists, and other PDA functions. The system can optionally include a speaker and microphone voice module, wherein the processor is programmed to process data to permit voice-over-internet protocol (IP) transfer. Also helpful, the program can permit alarm silencing of a bedside monitor, bedside admitting and discharging of patients, and adjustment of alarm parameter violation limits.

Various other features, objects and advantages of the present invention will be made apparent from the following detailed description and the drawings, in which:
Fig. 1 is a schematic diagram of a prior art one-way wireless clinical information management system.
Fig. 2 is a schematic diagram of a bi-directional, wireless clinical information management system according to the present invention.
Fig. 3 is a block diagram of a network connecting the clinical information management system of Fig. 2.
Fig. 4 is a block diagram of a portable patient monitoring device in accordance with present invention.
Fig. 5 is a flow chart of a process and software in accordance with the present invention.

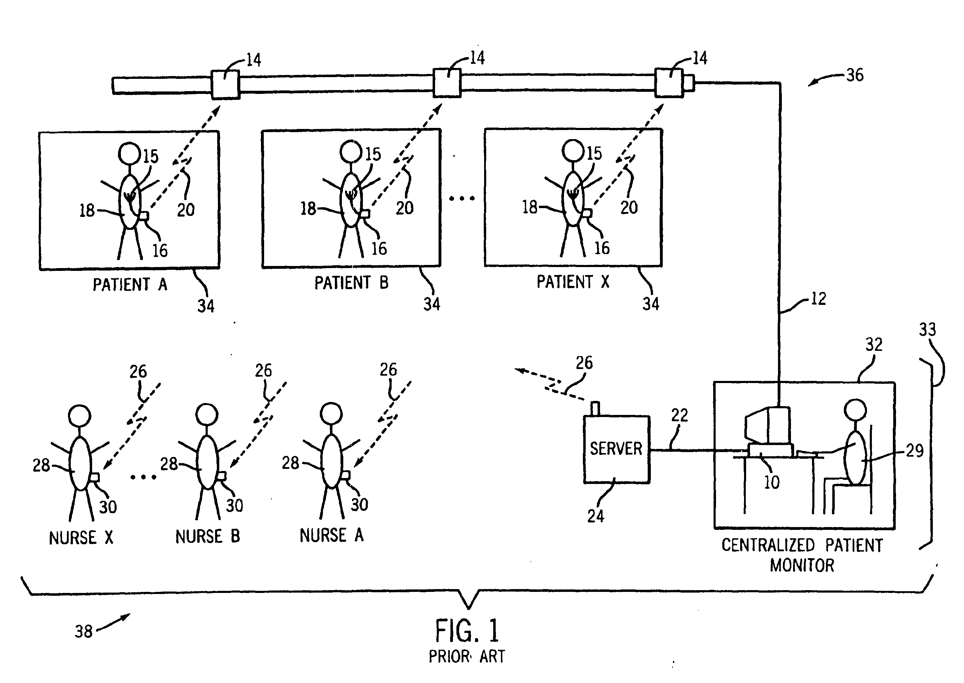

Fig. 1 is a prior art clinical information management system 36 that relays telemetry signals 20, such as patient vital signs and waveforms, from a plurality of patients 18 in patient rooms 34 to a plurality of mobile health care providers 28, for use in a health care facility 38. A centralized patient monitor (CPM) 32 includes a computer 10 staffed by a health care provider 29 in the vicinity of a central monitoring point such as a nurses' station 33. The CPM 32 is connected via a communications link 12 to a series of telemetry receivers 14 for each of a plurality of patient telemetry transmitters 16. The telemetry transmitters 16 are carried by patients 18 and send a one-way, wireless signal 20 to the receivers 14 from ECG leads 15. The receivers 14 then relay the signals 20 to the CMP 32. The CPM 32 is also connected via a communications link 22 to a server 24 that stores and relays data. The server 24 relays signals 26 to the mobile health care providers 28 who carry a receiving device 30 to display the signal information 26.

For instance, if an irregularity initiates an alarm sequence on a bedside patient monitor (not shown), the roaming health care provider 28 can be notified regardless of his or her position in the hospital, or other such health care facilities 38. In addition, the patient 18 gains the ability to move freely by use of a wireless link between the patient telemetry transmitter 26 and cumbersome stationary bedside monitoring systems. Although this system increases patients' 18 and attendants' 28 roaming capability, it retains rigid one-way communication abilities and on-site patient care requirements.

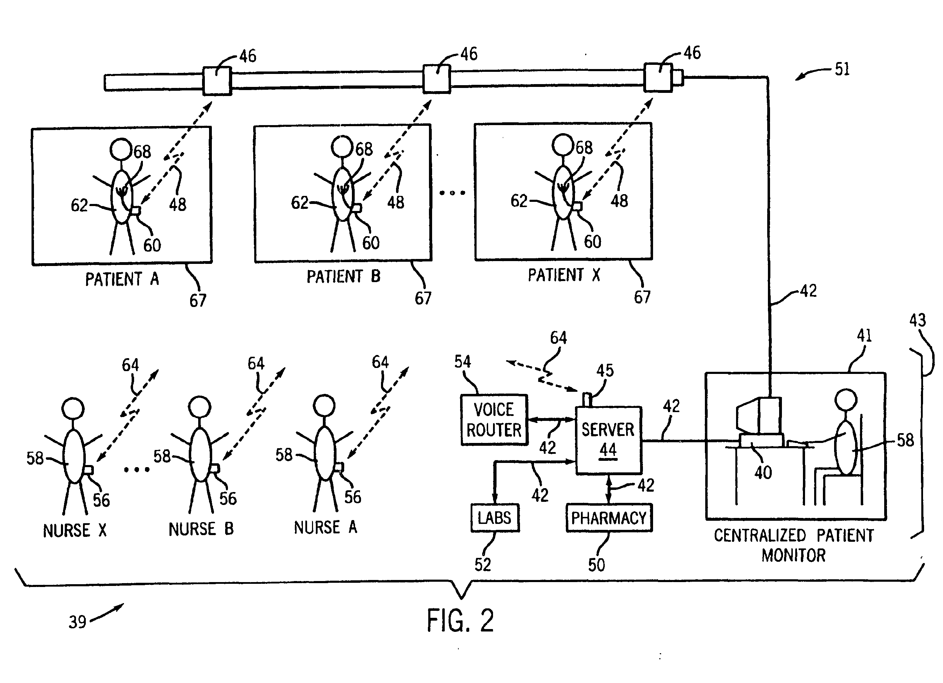

Fig. 2 is a block diagram of the present invention. A clinical patient information management system 51 according to the present invention includes a centralized patient monitor CPM 41 connected by a communications link 42 to a server 44 to store and retrieve patient data from patients 62 in patient rooms 67. The CPM 41 also is linked to a plurality of telemetry receivers 46 to relay telemetry transmitter signals 48 through the common network 42. The CPM 41 optionally includes a computer terminal 40 operated by a health care provider 58 within a central location such as a central nurses' station 43 in a health care facility 39. However, with implementation of the present invention, as will become apparent, the health care provider 58, positioned at nurses' station 43, can be eliminated since the mobile health care providers 58 will now have the data and control previously reserved for the nurse in the centralized patient monitor 41. The server 44 is connected peripherally to hospital labs 52, a pharmacy 50, a voice router 54, and to a number of portable patient monitoring devices (PPMs) 56 by a wireless local area network (WLAN). Simultaneously, the server 44 can access real time data from labs 52 and the pharmacy 50, and can transmit such data to the PPMs 56, keeping the health care providers 58 updated at remote locations. The server 44 can also convey signals from the CPM 41 to the PPMs 46. Such signals can include current patient data, ECG waveforms, and alarm signals.

The PPMs 56 are carried by the mobile health care providers 56 and are connected by bi-directional, wireless communication 64 to the server 44. WLAN access points 45 are connected to the server 44 to relay signals 64 between the server 44 and the plurality of PPMs 56. WLAN access points 45 include commercially available two-way modem technology transmitters/receivers and antennas that preferably operate on an industry standard protocol. PPMs 56, positioned on mobile health care providers 58, receive signals 64 from the WLAN access points 45 and display them in clear, comprehensive, and user-friendly form. The two-way wireless signals 64 are transmitted between the server 44 via the WLAN 45 and the health care providers 58 to enhance the caregivers' mobility.

Wireless patient telemetry transmitters/receivers (i.e., transceivers) 60 are carried by patients 62 and accord patients 62 with a degree of freedom and flexibility to accelerate their recovery. While vital sign signals 48 that are captured by the ECG leads 68 are normally displayed on stationary bedside monitoring equipment, with wireless telemetry transceivers 60, patients 62, can get exercise while ECG leads 68 monitor vital signs and send the signals 48 to the PPMs 56. This allows health care providers 58 to maneuver and obtain the clinical information without bulky monitoring systems.

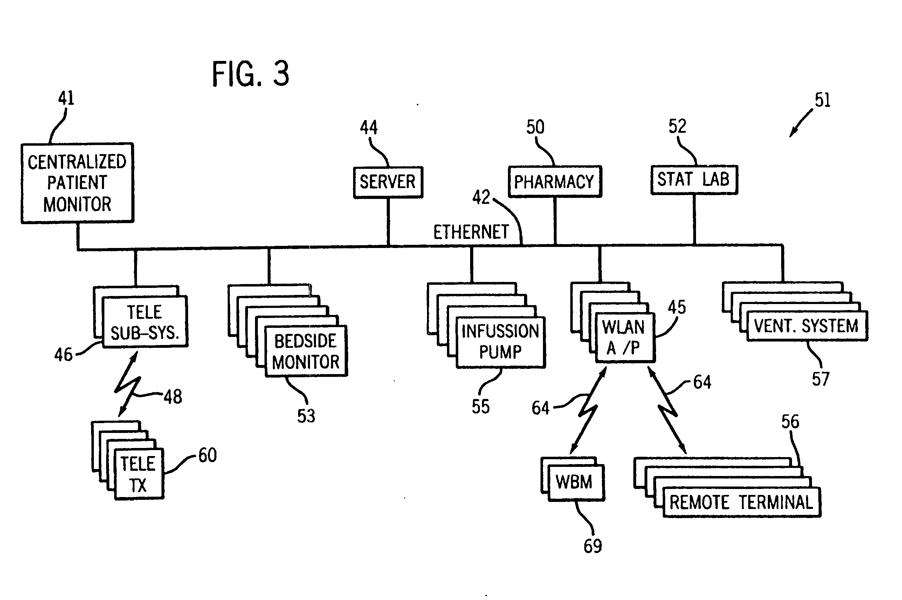

Fig. 3 shows a block diagram of the infrastructure for a clinical patient information monitoring system 51 connected through a network, such as an Ethernet 42. In a preferred embodiment, the network 42 is a GE Marquette Unity™ Ethernet network available from GE Medical Systems Information Technologies, Inc. The network 42 utilizes IEEE 802.3 standard Ethernet protocols and IEEE 802.11 WLAN as an extension of the wired system to network devices such as ventilators 57, infusion pumps 55, bedside patient monitoring systems 53, telemetry systems 46, and other hospital information systems in a comprehensive, efficient manner. The network 42 allows multiple devices connected to the network to operate in synchronization with each other while distributing patient information among the various hospital information systems. The pharmacy 50, labs 52, WLAN access points 45, the CPM 41, server 44, and portable patient monitors 56 comprise various hospital information systems.

In a preferred embodiment of the invention, life-sustaining devices such as ventilation systems 57 and infusion pumps 55, along with bedside monitors 53, are networked through network 42 to communicate with server 44 and CPM 41. Pharmacy 50 and labs 52 information systems provide data to health care providers 58 through the server 44, WLAN access points 45, patient telemetry systems 46, and the portable patient monitors 56 over the network 42. Optionally, a wireless patient bedside monitor 69 can operate within the clinical information management system 51. In that case, the signals 64 are also transmitted over the WLAN access points 45 to the wireless bedside monitor 69.

Other networked systems within the facility can interface with the clinical patient information monitoring system 51 through the use of standard based networking. Critical reports and diagnostic analysis prepared in various regions of the facility are available as they are completed by direct interfaces between the clinical patient information monitoring system and the decentralized diagnostic locations. The clinical patient information management system 51 also provides a decentralized, wireless, real-time monitoring capability for infusion pumps 55, ventilators 57, and other potentially non-networked machines such as the bedside monitors 53, the wireless bedside monitors 69, etc.

In a preferred embodiment, the wireless patient telemetry transceivers 60 transmit data 48 to the telemetry receivers 46 that then relays the information to appropriate appendages of the network 42. Such appendages include the PPM remote terminals 56. Any required modification in patient care parameters by a health care provider 58 can be relayed back to the patient through the network 42 to control patient care by using the PPMs 56. In response to patient status, a two-way wireless connection 64 between the server 44 and the PPMs 56 allow the health care provider 58 to adjust patient care parameters and/or to modify alarms. The health care provider 58 has the advantage in this system of the ability to send and receive information across the two-way wireless connection 64. For example, the health care provider 58 is able to compare dosage instructions on prescription medicine from the pharmacy 50, adjust alarm parameters, communicate through a voice module 72, and monitor real-time patient information such as ECG waveforms. The two-way wireless remote nature of the system 51 provides the health care provider 58 with a way to achieve more accurate and efficient care.

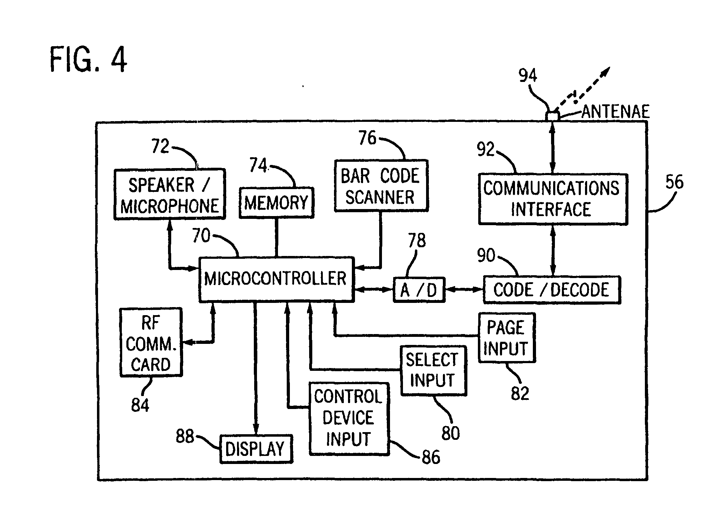

Fig. 4 is a block diagram of a PPM remote terminal 56 according to the present invention. The PPM 56, in general, has a size and shape that allows health care providers to carry it for extended periods of time. At the hardware level of a preferred embodiment, the PPM is based on a Personal Data Assistant (PDA) platform. The PDA provides a direct interface to the user with the various functions of the clinical information management system, in addition to personal efficiency functions such as a calendar, to-do lists, reminders, e-mail, and other such functions. The PPM is also designed to record voice reports to enable immediate recordation of patient events. Preferably, the invention could support dictation functions to record patient medical events. In one embodiment of the invention, the PDA can be adapted from a commercially available device, such as Symbol Technologies SPT 1700. The device can be based on the Palm Computing® platform, the Windows CE® platforms, or any other comparable or similar platform. Windows CE® is a registered trademark of Microsoft Corp.

In the preferred embodiment, the PPM 56 includes a microcontroller 70 connected to a speaker/microphone voice module 72 to receive and transmit voice data. A memory unit 74 is preferably a combination of ROM and RAM, wherein the ROM is used for static data, such as computer programs, and the RAM is used for dynamic data, such as the ECG signals received from the patient 62. A bar code scanner 76 is provided to read bar codes, such as those used to identify patients and those used on reports and pharmaceutical products. An A/D converter 78 converts analog to digital data for processing by the microcontroller 70, and conversely converts the digital data from the microcontroller to analog form which is then supplied to encoder 90 to code and decode the analog data for transmission through the communications interface 92 and an antenna 94. An input select key 80 is provided to select which particular menu is to be displayed on display 88. A control device input 86 is used to navigate through each of the menus that are displayed. Page input 82 is provided to acknowledge a page to a particular health care provider. An RF communications card 84 is connected to the microcontroller 70 which can include an industry standard PCMCIA card for RF communication. These components, in the aggregate, achieve functions that enable the health care provider to administer efficient and accurate care.

More specifically, the microcontroller 70 is programmed to receive and process patient data, display the data, and receive and transmit care parameters via the ancillary devices connected to the microcontroller 70. The microcontroller 70 is programmed to receive and transmit patient data in conjunction with the PPM communication interface 92. Some of the data contained includes bar code data that the microcontroller 70 is programmed to receive from the bar code scanner 76. The microcontroller 70 is also programmed to receive and transmit audio data from a speaker/microphone module 72, and to display information through the display 88 of the PPM 56. The health care provider 58, by utilizing attributes of the PPM 56, can input selections that the microcontroller 70 is programmed to receive.

The multiple functions of the PPM 56 include devices that interface with the microcontroller 70, and utilize capabilities of the clinical patient information system 51. Audio data received and transmitted by the microcontroller 70 from the audio module 72 is possible through "voice over IP" protocols that support transmitting compressed voice data over an Ethernet. The Ethernet network 42, in conjunction with the PPM 56, supports telephony and paging functions anywhere within the coverage area. No additional RF infrastructure is required to attain telephony or paging services. This solution eliminates the need for a health care provider 58 to carry multiple devices throughout the day such as mobile phones and/or pagers. Additionally, the use of a single RF protocol reduces the likelihood of interference from other wireless systems.

Automated data entry and retrieval via an integrated bar-code scanner 76 further involves multiple aspects of the clinical information management system 51. Information encoded on patient wristbands and pharmaceutical barcodes is acquired by the bar code scanner 76 and processed by the microcontroller 70. The microcontroller 70 compares the corresponding data from a centralized database maintained by the pharmacy 50, and standing doctors' orders contained in the patient record, for prescription and dosage accuracy.

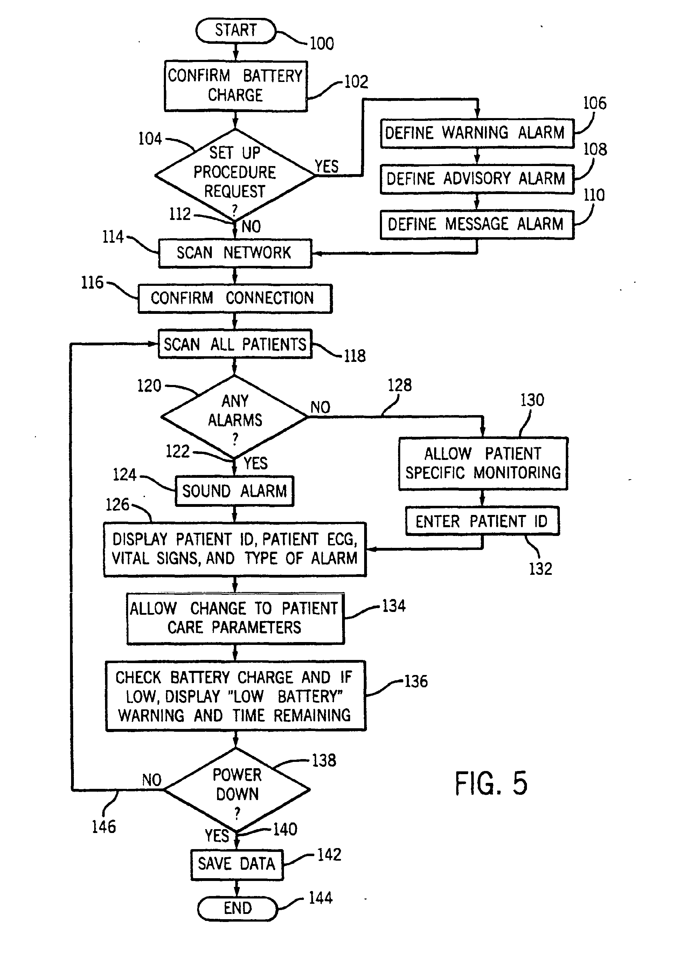

In a typical preferred embodiment of this invention the microcontroller 70 is programmed to interact with components of the PPM 56 to allow the health care provider 58 to communicate on a two-way basis with other segments of the clinical information management system 51. The preferred implementation sequence of the present invention is expressed by the flow chart of Fig. 5.

At the start 100 of the software sequence of the PPM 56 the battery charge is checked at 102. In accordance with the invention, a setup procedure request is initiated 104 if the battery charge is sufficient for operation. If desired at this point, the health care provider can access alarm-warning parameters and define warnings 106, advisories 108, and messages 110. If not desired 112, the routine commences scanning the network 114.

Initially, the scanning purpose is to check the validity of the network connection 116. The next scan is of the patients on the system 118. The health care provider is primarily concerned with current alarm flags that would require instant attention. If there are alarms 120, 122, the PPM will sound an audio alarm 124 through the audio module, and patient information is displayed 126. If there are no alarms 120, 128, then the health care provider enters a subroutine to monitor specific patients 130. After entering a patient ID 132, the subroutine joins with the main routine to display pertinent information, such as patient ID, ECG data, vital signs, and alarm type at 126. The health care provider can then decide to change any of the patient parameters 134, including turning alarms off.

During specific patient monitoring, the health care provider can scan barcode IDs that are then compared by the microcontroller with information gleaned from hospital lab and pharmacy data. This data is compared in real time with the earliest data available. In the background, the microcontroller is programmed to periodically check the battery charge 136. There are several stages of alert for the battery charge, ranging from 30-minute charge warnings, to urgent five-minute warnings. The health care provider can power down the PPM 138, 140 at the end of a shift which then automatically triggers a save function for all the data 142, and the sequence ends at 144. If the battery charge is sufficient for continued use and the health care provider desires continued use 138, 146, patient scanning and monitoring continues until the device is powered down 138, 140.

As previously discussed, the portable patient monitor (PPM) is preferably packaged within a housing that is transportable on a health care provider for extended periods. Preferably, the PPM has an approximate length of 7" (17.8 cm), a width of approximately 3.75" (7.5 cm) and a thickness of approximately 1.0" (2.54 cm).

## Claims

1. A mobile clinical information management system (42) to decentralize patient monitoring comprising:
a plurality of wireless bi-directional portable patient monitors (56) that are transportable on a health care provider (58) for extended periods, each portable patient monitor (56) comprising:
a communication interface (92) to receive patient data (64) from a wireless local area network (WLAN) within a medical care facility (39) and transmit care parameters (64) as needed to the WLAN in response thereto;
a processor (70) connected to the communication interface (92) to process the patient data (64) and the care parameters (64), wherein the processor (70) is programmed to allow adjustment (134) of alarm parameter violation limits;
a display (88) connected to the processor (70) to display the processed patient data (64) in human discernable form; and
an input device (86) connected to the processor (70) to allow a change in the care parameters (64) by a health care provider (58);
the portable patient monitors (56) having a configuration to allow wireless transport on a health care provider (58) for extended periods; and the system further comprising:
a plurality of bedside patient monitors (53, 69) to connect to a plurality of patients (62) and transmit patient data (48); and
the WLAN coupled to the plurality of bedside patient monitors (53, 69) and the portable patient monitors (56), and wherein said WLAN operates according to a single RF protocol; **characterized in that**
each portable patient monitor (56) is assigned to a given number of patients (62); and
each portable patient monitor (56) further comprises a bar code scanning module (76) and a bar code scanner (76), and wherein the processor (70) is programmed to receive and compare patient data (64) with data obtainable from a centralized database (50) that includes pharmaceutical and patient bar codes to ensure dosage accuracy, and doctor orders.

2. The system (41) of claim 1 wherein each processor (70) decodes (90) the patient data (64) to process and display (88) the patient data (64) and encodes (90) the care parameters (64) to transmit the care parameters (64) to the WLAN.

3. The system (41) of claim 1 wherein each processor (70) processes the patient data (64) to display ECG and vital sign data (48) for a selected patient (62).

## Patentansprüche

1. Mobiles klinisches Informationsverwaltungssystem (42), um Patientenüberwachung zu dezentralisieren, aufweisend:
mehrere drahtlose bi-direktionale tragbare Patientenüberwachungsgeräte (56), die für längere Zeiträume von einem Leistungserbringer (58) medizinischer Dienstleistung getragen werden können, wobei jedes tragbare Patientenüberwachungsgerät (56) aufweist:
eine Kommunikationsschnittstelle (92), um Patientendaten (64) aus einem drahtlosen lokalen Netz (WLAN) innerhalb einer medizinischen Betreuungseinrichtung (39) zu empfangen und Betreuungsparameter (64) nach Bedarf an das WLAN in Antwort darauf zu senden;
einen mit der Kommunikationsschnittstelle (92) verbundenen Prozessor (70), um die Patientendaten (64) und die Betreuungsparameter (64) zu verarbeiten, wobei der Prozessor (70) dafür programmiert ist, eine Anpassung (134) von Alarmparameter-Verletzungsgrenzwerten anzupassen;
eine mit dem Prozessor (70) verbundene Anzeigeeinrichtung (88), um die verarbeiteten Patientendaten (64) in von Menschen erkennbarer Form anzuzeigen; und
eine mit dem Prozessor (70) verbundene Eingabevorrichtung (86), um eine Änderung in den Betreuungsparametern (64) durch einen Leistungserbringer (58) medizinischer Dienstleistung (58) zu ermöglichen;
wobei die tragbaren Patientenüberwachungsgeräte (56) eine Konfiguration haben, um einen drahtlosen Transport an einem Leistungserbringer (58) medizinischer Dienstleistung (58) für längere Zeiträume zu ermöglichen; und das System ferner aufweist:
mehrere bettseitige Patientenüberwachungsgeräte (53, 69), zum Verbinden mit mehreren Patienten (62) und zum Übertragen von Patientendaten (48); und
das mit den mehreren bettseitigen Patientenüberwachungsgeräten (53, 69) und den tragbaren Patientenüberwachungsgeräten (56) gekoppelte WLAN, und wobei das WLAN mit nur einem HF-Protokoll arbeitet; **dadurch gekennzeichnet, dass**
jedem tragbaren Patientenüberwachungsgerät (56) eine vorgegebene Anzahl von Patienten (62) zugewiesen ist; und
jedes tragbare Patientenüberwachungsgerät (56) ferner ein Balkencode-Scanmodul (78) und ein Balkencode-Scanner (76) aufweist, und wobei der Prozessor (70) dafür programmiert ist, Patientendaten (64) zu empfangen und mit Daten zu vergleichen, die von einer zentralen Datenbank (50) abrufbar sind, die pharmazeutische und Patienten-Balkencodes enthält, um die Dosierungsgenauigkeit und Arztanweisungen sicherzustellen.

2. System (41) nach Anspruch 1, wobei jeder Prozessor (70) die Patientendaten (64) decodiert (90), um die Patientendaten (64) zu verarbeiten und anzuzeigen (88) und die Betreuungsparameter (64) codiert (90), um die Betreuungsparameter (64) an das WLAN zu übertragen.

3. System (41) nach Anspruch 1, wobei jeder Prozessor (70) die Patientendaten (64) verarbeitet, um EKG- und Vitalzeichen-Daten (48) für einen ausgewählten Patienten (62) anzuzeigen.

## Revendications

1. Système (42) mobile de gestion des informations cliniques pour décentraliser la surveillance des patients comprenant :
une pluralité de postes individuels (56) portables bidirectionnels sans fil qui sont transportables chez un prestataire (58) de soins de santé pendant des périodes prolongées, chaque poste individuel (56) portable comprenant :
une interface (92) de communication pour recevoir des données (64) de patient depuis un réseau local (WLAN) sans fil dans une unité (39) de soins médicaux et transmettre des paramètres (64) de soin tels que nécessaires au WLAN en réponse à celui-ci ;
un processeur (70) relié à l'interface (92) de communication pour traiter les données (64) de patient et les paramètres (64) de soin, le processeur (70) étant programmé pour permettre un réglage (134) des limites de violation des paramètres d'alerte ;
un écran (88) relié au processeur (70) pour afficher les données (64) de patient traitées sous une forme humainement discernable ; et
un dispositif (86) de saisie relié au processeur (70) pour permettre un changement dans les paramètres (64) de soin par un prestataire (58) de soins de santé ;
les postes individuels (56) portables ayant une configuration pour permettre le transport sans fil chez un prestataire (58) de soins médicaux pendant des périodes prolongées ; et le système comprenant en outre :
une pluralité de postes individuels (53, 69) de lit à relier à une pluralité de patients (62) et pour transmettre des données (48) de patient ; et
le WLAN couplé à la pluralité de postes individuels (53, 69) de lit et aux postes individuels (56) portables, ledit WLAN fonctionnant selon un simple protocole RF, **caractérisé en ce que**
chaque poste individuel (56) portable est affecté à un certain nombre de patients (62) ; et
chaque poste individuel (56) portable comprend en outre un module (76) de scannage de code-barres et un scanner (76) de code-barres, et le processeur (70) étant programmé pour recevoir et comparer des données (64) de patient avec des données pouvant être obtenues depuis une base (50) de données centralisée qui contient des codes-barres pharmaceutiques et de patients pour assurer une exactitude de dosage, et des ordres du médecin.

2. Système (41) mobile selon la revendication 1, **caractérisé en ce que** chaque processeur (70) décode (90) les données (64) de patient pour traiter et afficher (88) les données (64) de patient et coder (90) les paramètres (64) de soins pour transmettre les paramètres (64) de soin au WLAN.

3. Système (41) mobile selon la revendication 1, **caractérisé en ce que** chaque processeur (70) traite les données (64) de patient pour afficher des données (48) d'électrocardiogramme et de signe vital pour un patient sélectionné (62).
